# EUROPEAN PATENT APPLICATION

(11) **EP 2 422 764 A1**
(43) Date of publication of application: **29.02.2012**
(21) Application number: 10767202.4
(22) Date of filing: 23.04.2010
(51) Int. Cl.: A61K 8/97, A23G 3/00, A23G 3/34, A61K 36/18, A61P 1/02, A61Q 15/00

(54) **COMPOSITION FOR USE IN THE ORAL CAVITY**

(30) Priority: 23.04.2009 JP 2009105121
(71) Applicant: Lotte Co., Ltd., Tokyo 160-0023 (JP)
(72) Inventor: SHIZUKUISHI, Satoshi, Suita-shi Osaka 565-0871 (JP); TANAKA, Muneo, Suita-shi Osaka 565-0871 (JP); SHIMIZU, Katsumasa, Saitama-shi Saitama 336-0027 (JP); OSAWA, Kenji, Tokyo 160-0023 (JP)
(74) Representative: Blodig, Wolfgang
(86) International application number: PCT/JP2010/057728
(87) International publication number: WO 2010/123155

(57) **Abstract**

Provided is an oral composition having a tongue coating removing effect and a strong bad breath removing effect. An oral composition having a tongue coating removing action, **characterized by** containing an extract of eucalyptus, a plant of the Myrtaceae family, as an essential ingredient.

## Description

### TECHNICAL FIELD

The present invention relates to a tongue coating removing effect in the mouth by continuously consuming a chewing gum incorporating a eucalyptus extract.

### BACKGROUND ART

A eucalyptus extract has an antibacterial activity on various periodontal disease bacteria and especially has a high antibacterial activity on Porphyromonas gingivalis, which is a typical periodontal disease bacterium. In addition, recent studies have clarified that as the antibacterial activity, macrocarpals in a eucalyptus extract inhibit the proteolytic enzyme produced by the bacterium and inhibit the ability of the bacterium to adhere to hydroxyapatite beads. For the above reasons, it is considered that a eucalyptus extract is a promising material for the prevention of periodontal diseases.

However, it has not yet been known that a eucalyptus extract has a tongue coating removing action.

The deodorizing action of a eucalyptus extract has been conventionally studied (Japanese Patent Application Laid-Open No. 2003-335647, Japanese Patent Application Laid-Open No. H05-161697, Japanese Patent Application Laid-Open No. H05-161696 and Japanese Patent Application Laid-Open No. S60-261458). Japanese Patent Application Laid-Open No. 2003-335647 discloses a deodorizing agent of a eucalyptus extract against allyl methyl sulfide, Japanese Patent Application Laid-Open No. H05-161697 discloses essential oils of plants of the Myrtaceae family as a deodorizing agent against the odor of methyl mercaptan (but no descriptions of eucalyptus (Myrtaceae) are shown), Japanese Patent Application Laid-Open No. H05-161696 discloses essential oils of plants of the Myrtaceae family as a deodorizing agent against the odor of ammonia (but no descriptions of eucalyptus are shown), and Japanese Patent Application Laid-Open No. S60-261458 discloses a deodorizing agent of an extract obtained from eucalyptus against ammonia, amines, mercaptans and nicotine.

Further, there are many reports on the antibacterial activity of a eucalyptus extract on various periodontal disease bacteria (Journal of Oral Science, 40 (3), 115-117 (1998); Journal of Dental Health, 53, 585-591 (2003); J. Nat, Prod, 59(9), 823-827 (1996); Natural Medicines, 52(1), 32-37 (1998); and J. Periodontol, 79(8), 1378-1385 (2008)). Journal of Oral Science, 40 (3), 115-117 (1998) discloses the inhibitory effect on plaque formation of a Fukuronori extract and a eucalyptus extract contained in a chewing gum, Journal of Dental Health, 53, 585-591 (2003) discloses the antibacterial activity of a eucalyptus leave extract on periodontopathic bacteria, J. Nat, Prod, 59(9), 823-827 (1996) discloses the antibacterial action of macrocalpals H, I and J obtained from eucalyptus leaves, Natural Medicines, 52(1), 32-37 (1998) discloses the antibacterial activity and glucosyl transferase inhibitory effect of Eucalyptus globulus on the bacteria in the oral cavity, and J. Periodontol, 79(8), 1378-1385 (2008) discloses the effect of a chewing gum incorporating a eucalyptus extract on periodontal health.

In addition, as the literature which discloses the removal of tongue coating, there are Japanese Patent Application Laid-Open No. 2005-281230, WO Publication No. 2003-090704, Japanese Patent Application Laid-Open No. 2000-178154, Japanese Patent Application Laid-Open No. H09-25221, Japanese Patent Application Laid-Open No. H10-182387 and Journal of Dental Health, 56, 37-41 (2006). Japanese Patent Application Laid-Open No. 2005-281230 is to physically remove tongue coating and discloses that tongue coating is physically removed to continually reduce bad breath by allowing a solid substrate containing insoluble natural plant fibers to act on the tongue. WO Publication No. 2003-090704, Japanese Patent Application Laid-Open No. 2000-178154, Japanese Patent Application Laid-Open No. H09-25221, Japanese Patent Application Laid-Open No. H10-182387 and Journal of Dental Health, 56, 37-41 (2006) are to chemically remove tongue coating. WO Publication No. 2003-090704 discloses a tongue coating removing composition having a tongue coating removing action (protease (actinidine)), Journal of Dental Health, 56, 37-41 (2006) discloses that as a result of comparing the amount of tongue coating before and after the use of a tablet incorporating protease (actinidine) and a placebo agent, the amount of tongue coating adhering was significantly reduced in the group using the protease-containing tablet, Japanese Patent Application Laid-Open No. 2000-178154 discloses a tongue coating removing agent characterized by containing sodium hydrogen carbonate, sodium carbonate, ethyl alcohol and a cationic bactericide and substantially no anionic surfactants and having a content of a nonionic surfactant of 0 to 0.2% by weight, Japanese Patent Application Laid-Open No. H09-25221 discloses a tongue coating removing agent comprised of an enzyme selected from the group consisting of N-acetylmuramidase, mutanolysin, lysozyme, levanase and lipase and a surfactant selected from the group consisting of a betaine type surfactant, an N-acyltaurine type surfactant, a methyl glucosyl ester type surfactant, a dialkyl sulfosuccinate type surfactant and a monoacyl phosphate type surfactant, and Japanese Patent Application Laid-Open No. H10-182387 discloses a tongue coating removing agent comprised of a linear condensed phosphate compound having a specific structure and/or a cyclic condensed phosphate compound having a specific structure.

As mentioned above, although many studies on a eucalyptus extract and removal of tongue coating have been performed, there are no reports which find that a eucalyptus extract has a tongue coating removing action.

### DISCLOSURE OF THE INVENTION

An object of the present invention is to provide a food and drink product and an oral composition, which contain a eucalyptus extract and have an excellent removing effect on tongue coating.

Tongue coating shows a coated state formed by the accumulation of food residues, desquamated epithelia, bacteria, blood cells and the like. It is known that the accumulation of tongue coating causes bad breath, dysgeusia and aspiration pneumonia, and the importance of taking care of the tongue has been reported. Consequently, the present study clarified that the amount of tongue coating was reduced by continuously consuming a gum incorporating a eucalyptus leave extract and physiological bad breath was also reduced.

That is, the present invention relates to an oral composition having a tongue coating removing action, characterized by containing an extract of eucalyptus, a plant of the Myrtaceae family, as an essential ingredient.

The present invention can also use an extract obtained from leaves of Eucalyptus plants (raw or dried) of the Myrtaceae family. In view of the improvement in the extraction rate, dried leaves are suitable.

A method for obtaining an extract of the above plant, which is an active ingredient of the present invention, is not particularly limited, but the above plant is pulverized by a suitable pulverization unit and an extract is prepared by a method of solvent extraction including two-step extraction and the like. As the extraction solvent, water, lower alcohols such as methanol, ethanol, n-propanol and n-butanol and organic solvents such as ether, chloroform, ethyl acetate, acetone, glycerin and propylene glycol are used singly or as mixtures of two or more, but water or a hydrophilic organic solvent is preferably used.

Furthermore, considering that the extract of the present invention is frequently used for humans or as food and drink, as the extraction solvent, a combination of water and ethanol is preferably used from the viewpoint of safety.

As the extraction conditions, the extraction can be performed at any of high temperature, room temperature and low temperature, but preferably performed at 50 to 90°C for approximately 1 to 5 hours. The resulting extract may be filtered and then concentrated or lyophilized under reduced pressure after distilling off the extraction solvent. In addition, these extracts are fractionated and purified using an organic solvent, column chromatography and the like, and the resulting extracts can also be used.

Further, the present invention relates to an oral composition wherein the oral composition is confectionery such as a chewing gum, a candy, a tablet and a gummy jelly, a mouth spray and a mouthwash.

In addition, the present invention relates to the oral composition described above wherein the content of the eucalyptus extract in the oral composition is 0.001% by weight or more and 10.0% by weight or less.

Further, the present invention relates to an oral composition having a preventive action on bad breath, characterized by containing an extract of eucalyptus, a plant of the Myrtaceae family, as an essential ingredient.

The present invention can be used for an oral composition such as confectionery such as a chewing gum, a candy, a tablet and a gummy jelly, a mouth spray and a mouthwash.

### BRIEF DESCRIPTION OF THE DRAWINGS

FIG. 1 is a graph showing change in ΔPLI with time.
FIG. 2 is a graph showing change in ΔTCS with time.
FIG. 3 is a graph showing change in ΔGI with time.
FIG. 4 is a graph showing change in ΔBOP with time.
FIG. 5 is a graph showing change in ΔPPD with time.
FIG. 6 is a graph showing change in ΔCAL with time.
FIG. 7 is a graph showing change in ΔOLT with time.
FIG. 8 is a graph showing change in ΔTotalVSC with time.
FIG. 9 is a graph showing change in ΔSaliva Flow Rate with time.

### BEST MODES FOR CARRYING OUT THE INVENTION

In evaluating the ameliorating effect on periodontal disease in the case of consuming a chewing gum incorporating a eucalyptus extract for a long period of time, the present inventors have found an unexpected tongue coating removing effect in the confirmation study of the preventive effect on periodontal disease, and have completed the present invention. This effect significantly reduced physiological bad breath.

The specific study contents of the present invention will be described below.

### Examples

### (Example 1)

After the removal of essential oils from eucalyptus leaves (Eucalyptus globulus) by steam distillation, the dried leaves were pulverized and 100 ml of water was added to 10 g of the dried leaves powder, followed by extracting under reflux at 70 to 90°C for approximately one hour. Thereafter, the extraction liquid was filtered out and removed and 100 ml of a 60% by weight aqueous solution of ethanol was added to the resulting extraction residue, followed by extracting under reflux at 70 to 90°C for approximately one hour. The extract was filtered out and then lyophilized after removing the organic solvents to prepare a eucalyptus extract.

### (Example 2)

Study gum 1, study gum 2 and study gum 3 as a control were prepared by incorporating the materials shown in the following Table 1 according to the "xylitol" tablet gum for the basic formulation of study gums in this Example.

**Table 1, Incorporation of Study Gums**

| Raw Material | Study Gum 1 | Study Gum 2 | Study Gum 3 |
|---|---|---|---|
| Eucalyptus Extract (Example 1) | 0.3 | 0.2 | 0 |
| Xylitol | 45.0 | 45.0 | 45.0 |
| Maltitol | 33.0 | 33.0 | 33.0 |
| Gum Base | 14.0 | 14.0 | 14.0 |
| Flavor | 1.0 | 1.0 | 1.0 |
| Coloring Agents | 0.1 | 0.1 | 0.1 |
| Gum Arabic | Remaining Quantity | Remaining Quantity | Remaining Quantity |

### (Example 3)

### Study Contents

The above study gums 1, 2 and 3 were allowed to be consumed by males and females in their 20's, 30's and 40's paying attention to the gingival conditions as subjects, and the efficacy (anti-periodontal disease effect) and safety evaluations of long-term consumption of the gums were carried out as follows.

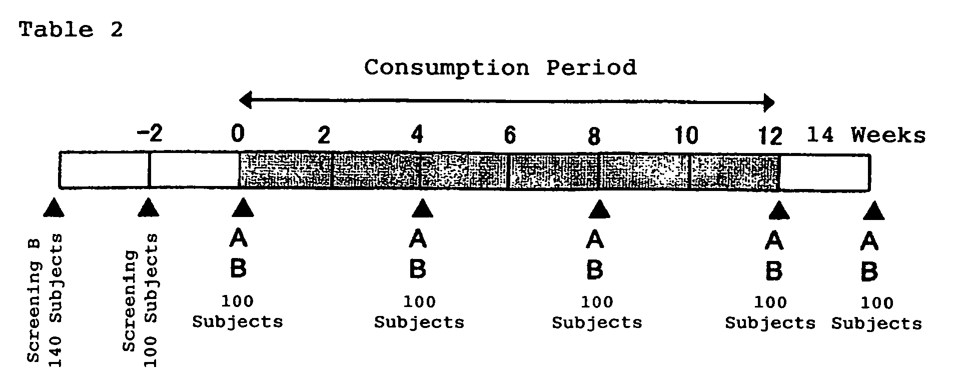

Evaluation item A: Gingival index (Gingival inflammation), plaque accumulation (adhesion of plaque), probing pocket depth (periodontal pocket depth), bleeding on probing, clinical attachment level, amount of tongue coating, number of bacteria in saliva, degree of bad breath, saliva flow rate, oral findings and questioning (induction of diarrhea)
Evaluation item B: Blood pressure, pulse, hematology, blood biochemistry, urinalysis
   1) The screening within the oral cavity and evaluation item B were performed for 149 males and females in their 20's to 40's who wanted to participate in the study. The study was started using 100 subjects who had no sites having a probing pocket depth of 6 mm or more and a mean value of the gingival index other than 0. Dental plaque and tartar were removed by oral cleaning (scaling) 2 weeks before the start of the study.
   2) By stratifying 100 subjects by age, sex and gingival index, the subjects were divided into 3 groups: a group consuming study gum 1 (high concentration group), a group consuming study gum 2 (low concentration group) and a group consuming study gum 3 (control group), and the subjects were allowed to consume 10 tablets (2 tablets × 5 times) a day for 12 weeks.
   3) At the baseline, week 4, week 8 and week 12 of the study and further at week 2 after completion of the study, the above evaluation items A and B were performed and significance test between groups was performed on each measurement item using statistical methods. Then, the anti-periodontal disease effect of the study gums and the safety evaluation of long-term consumption of the study gums were carried out. Since 4 subjects dropped out during the study period, the final analysis was performed for 96 subjects.
   4) For the within-group change with time, the comparison with week 0 was carried out by Dunnett test, the comparison with the control at each time point was carried out by Dunnett test, the efficacy of the drug used was analyzed by repeated measures analysis of variance (ANOVA) to investigate the interaction, and then the results were analyzed by Games-Howell test.

Here, the evaluation item A will be described.

### 1) Gingival index (GI)

Gingival index (GI) evaluation standards: (Lobene. R. R. et al.: J periodontal., 60, 1989) was used as an index. All teeth were evaluated, the periodontal site was divided into four sites (buccal side, tongue side, mesial side and distal side) and each site was scored using the following screening criteria.
0 = Normal
1 = Mild inflammation (slight change in color and change in tissue of the gingiva) is observed at part of the gingiva.
2 = Mild inflammation (slight change in color and change in tissue of the gingiva) is observed at the entire periphery of the gingiva.
3 = Moderate inflammation (moderate luster, redness, edema, swelling) is observed at the gingiva.
4 = Severe inflammation (obvious redness, edema, swelling, spontaneous bleeding, ulcer) is observed at the gingiva.

### 2) Plaque index (PLI)

The method of Suzuki et al. (Journal of Dental Health, 20 (3) 1971) was used. Six teeth: the upper right and lower left first molars, upper left and lower right premolars and upper left and lower right central incisors were evaluated. Each tooth was measured at the two points of the mesiodistal buccal corner, one point of the buccal center, two points of the lingual mesiodistal buccal corner and one point of the lingual center, i.e. 36 points in total were measured. The height of the plaque adhering from the gingival periphery was recorded in units of 0.5 mm using a probe.

### 3) Probing Pocket Depth (PPD)

All teeth were evaluated. Each tooth was measured at the two points of the mesiodistal buccal corner, one point of the buccal center, two points of the lingual mesiodistal buccal corner and one point of the lingual center. The distance from the gingival periphery to the pocket bottom was recorded in units of 0.5 mm using a probe.

### 4) Bleeding on probing (BOP)

When the gingival pocket was measured, the site in which bleeding from the gingiva was observed was recorded.

### 5) Clinical attachment level (CAL)

The distance from the cement-enamel border to the gingival periphery was measured using a probe and calculated together with the results of the gingival pocket depth.

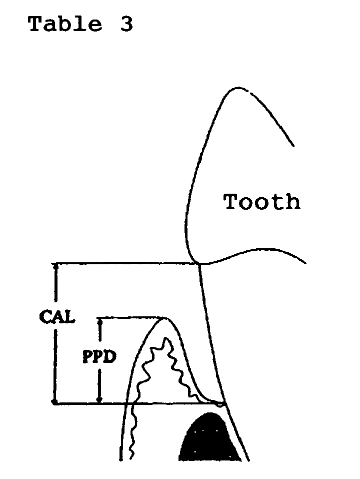

### 6) Amount of Tongue Coating

Tongue coating score (TCS): the area (0 to 3) × the thickness (0 to 2) was recorded.

### 7) Degree of Bad Breath

(I) Gas Chromatography Analysis: The concentration of the volatile sulfides (hydrogen sulfide, methyl mercaptan and dimethyl sulfide) contained in the gas in the mouth was measured by gas chromatography. The total concentration of hydrogen sulfide, methyl mercaptan and dimethyl sulfide was recorded in ppm as the total volatile sulfide concentration (Total VSC).
(II) Sensory Study: A plurality of examiners evaluated the breath exhaled into a scent bag using the following standards.

### Organoleptic test (OLT)

0: An odor higher than the odor threshold value is not felt
1: An odor higher than the odor threshold value is felt but cannot be recognized as a bad odor
2: An odor which can be recognized as a bad odor
3: An odor which can be easily considered a bad odor
4: A strong bad odor which is tolerable
5: A very strong odor which is intolerable

### 8) Evaluation of Saliva Flow Rate

The whole saliva, which was obtained by stimulating by chewing paraffin for 5 minutes, was collected and recorded in ml/min.

### 9) Oral Findings and Questionings (Induction of Diarrhea)

Oral abnormalities, that is, abnormalities of hard tissues (the presence or absence of abnormal decalcification or coloration of teeth) or abnormalities of soft tissues (abnormal stomatitis of the buccal mucosa, gingiva and tongue or the presence or absence of ulcer formation) were checked by ocular inspection. In addition, the presence or absence of diarrhea during the study period was inspected.

### (Results)

### 1. Comparison between groups of the amount of change in the clinical index during the study period

As a result of analyzing the comparison between groups of the amount of change in the clinical index during the study period, a significant interaction was observed in the plaque index, amount of tongue coating, gingival index, bleeding from the gingiva, probing pocket depth and organoleptic test, and a significant difference in both the low concentration group and the high concentration group was observed even for the comparison with the control group. However, no significant interaction was observed in the clinical attachment level, total volatile sulfide concentration and saliva flow rate.

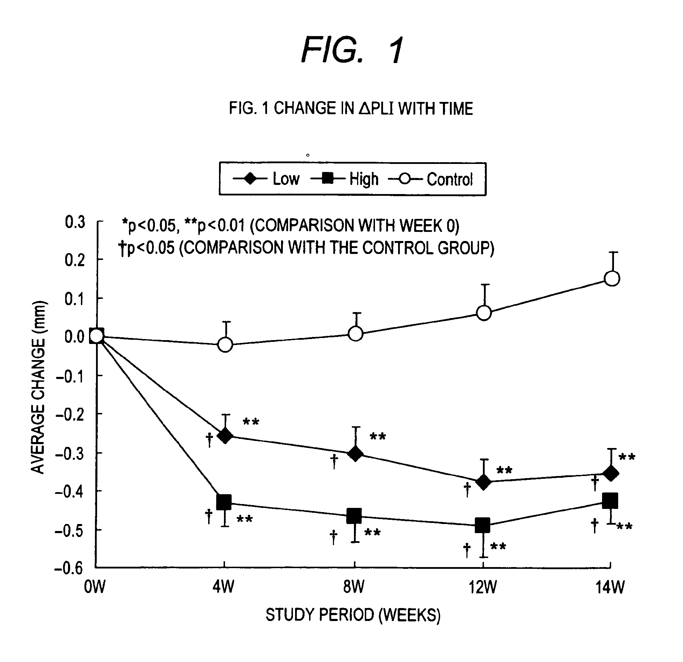

Here, the changes in each index with time are shown in FIGs. 1 to 9.

In the plaque index (FIG. 1), a significant reduction (p < 0.01) was observed at weeks 4, 8, 12 and 14 in both the low concentration group (Low) and the high concentration group (High), as compared with week 0. In addition, a significant difference (p < 0.05) was observed at week 4, 8, 12 and 14 in both the low concentration group and the high concentration group, as compared with the control group (Control).

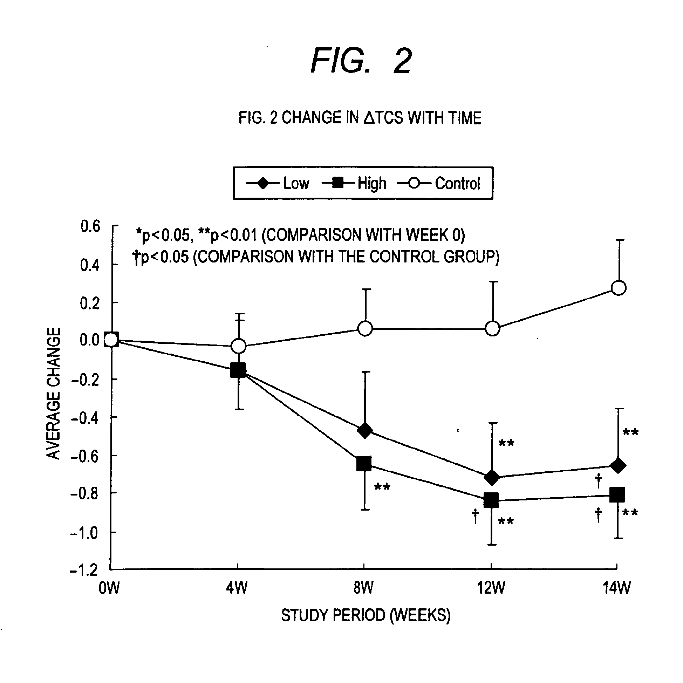

In the amount of tongue coating (FIG. 2), a significant reduction (p < 0.01) was observed at weeks 12 and 14 in the low concentration group and at weeks 8, 12 and 14 in the high concentration group, as compared with week 0. In addition, a significant difference (p < 0.05) was observed at week 14 in the low concentration group and at weeks 12 and 14 in the high concentration group, as compared with the control group.

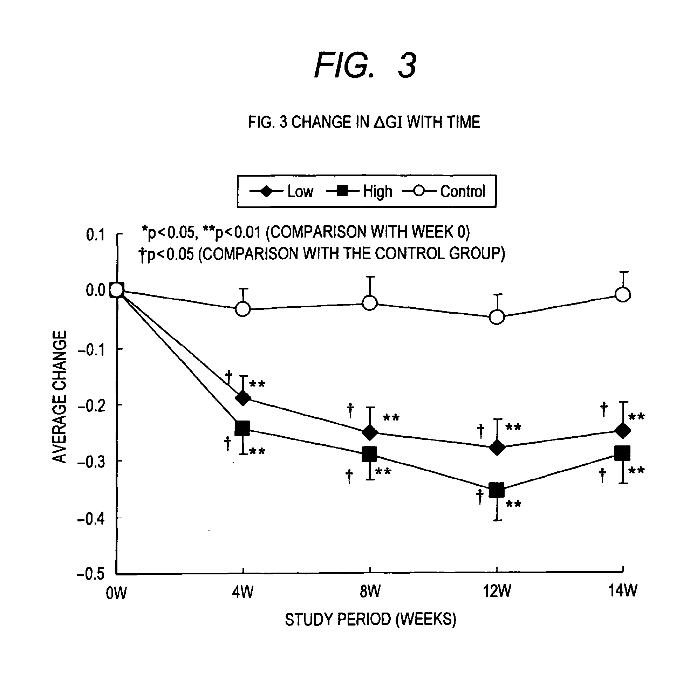

In the gingival index (FIG. 3), a significant reduction (p < 0.01) was observed at weeks 4, 8, 12 and 14 in both the low concentration group and the high concentration group, as compared with week 0. In addition, a significant difference (p < 0.05) was observed at weeks 4, 8, 12 and 14 in both the low concentration group and the high concentration group, as compared with the control group.

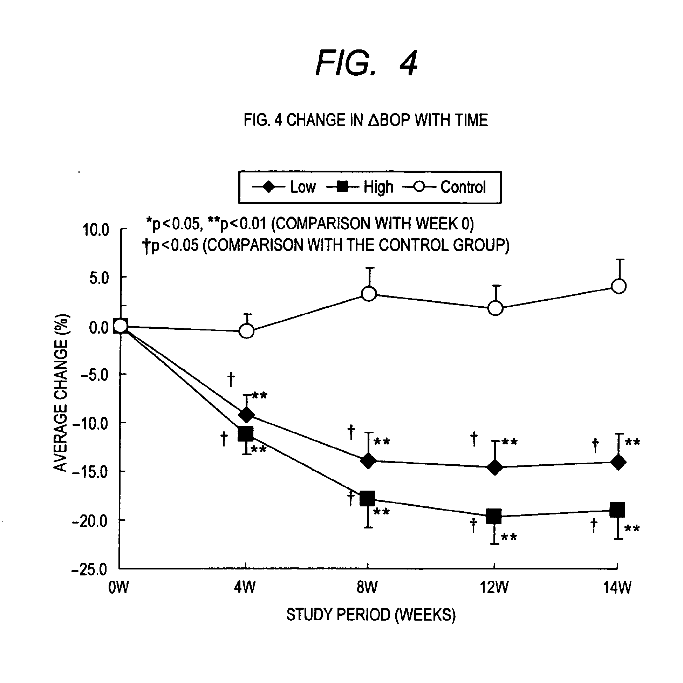

In the bleeding on probing (FIG. 4), a significant reduction (p < 0.05) was observed at weeks 4, 8, 12 and 14 in both the low concentration group and the high concentration group, as compared with week 0. In addition, a significant difference (p < 0.05) was observed at weeks 4, 8, 12 and 14 in both the low concentration group and the high concentration group, as compared with the control group.

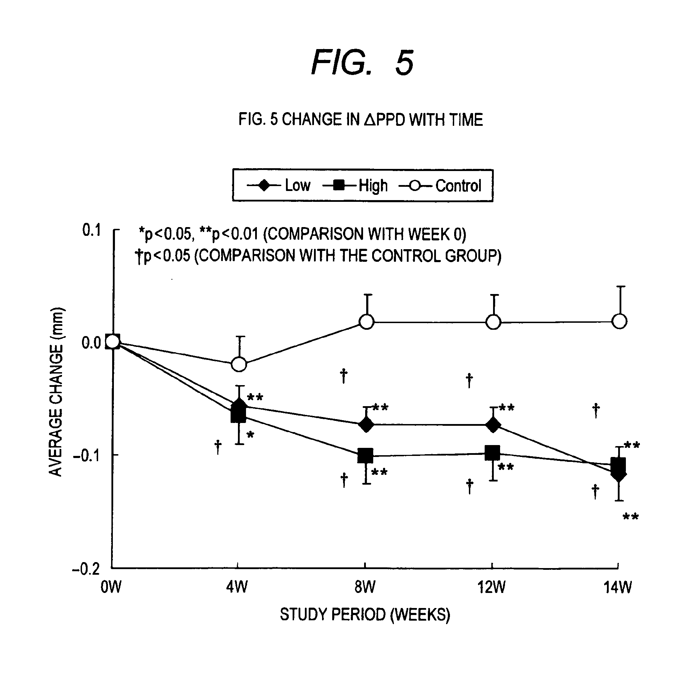

In the probing pocket depth (FIG. 5), a significant reduction (p < 0.01) was observed at weeks 4, 8, 12 and 14 in the low concentration group, as compared with week 0, and a significant reduction was observed at week 4 (p < 0.05) and at weeks 8, 12 and 14 (p < 0.01) in the high concentration group, as compared with week 0. In addition, a significant difference (p < 0.05) was observed at weeks 8, 12 and 14 in the low concentration group and at weeks 4, 8, 12 and 14 in the high concentration group, as compared with the control group.

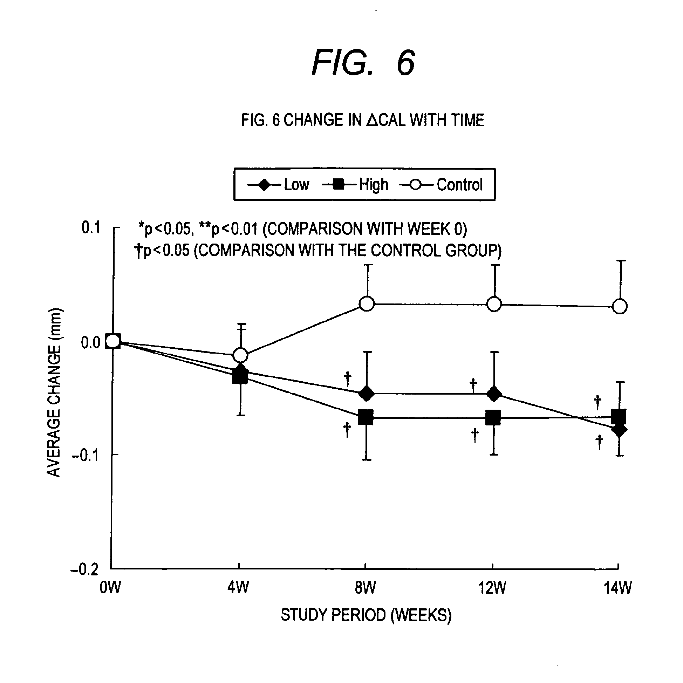

In the clinical attachment level (FIG. 6), no significant difference was observed at weeks 4, 8, 12 and 14 in all of the groups, as compared with week 0. In addition, a significant difference (p < 0.05) was observed at weeks 8, 12 and 14 in both the low concentration group and the high concentration group, as compared with the control group.

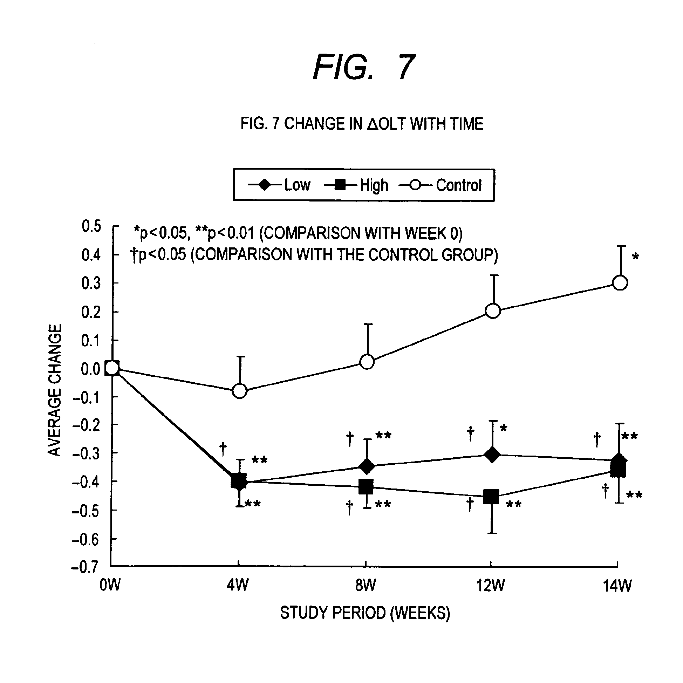

In the organoleptic test (FIG. 7), a significant reduction (p < 0.01, and p < 0.05 at week 12 only in the low concentration group) was observed at weeks 4, 8, 12 and 14 in both the low concentration group and the high concentration group, as compared with week 0. In addition, a significant difference (p < 0.05) was observed at weeks 4, 8, 12 and 14 in the low concentration group and at weeks 8, 12 and 14 in the high concentration group, as compared with the control group.

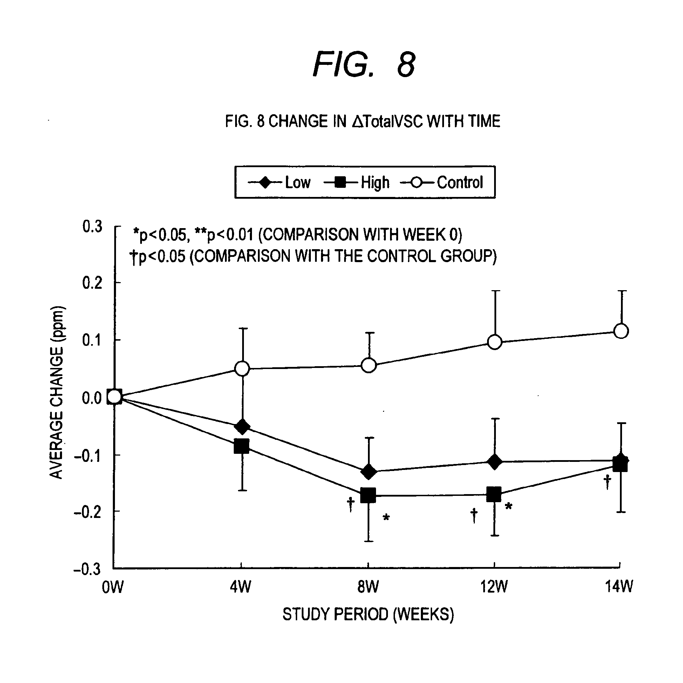

In the total volatile sulfide concentration (FIG. 8), a significant reduction (p < 0.05) was observed at weeks 8 and 12 in the high concentration group, as compared with week 0. In addition, a significant difference (p < 0.05) was observed at weeks 8, 12 and 14 in the high concentration group, as compared with the control group.

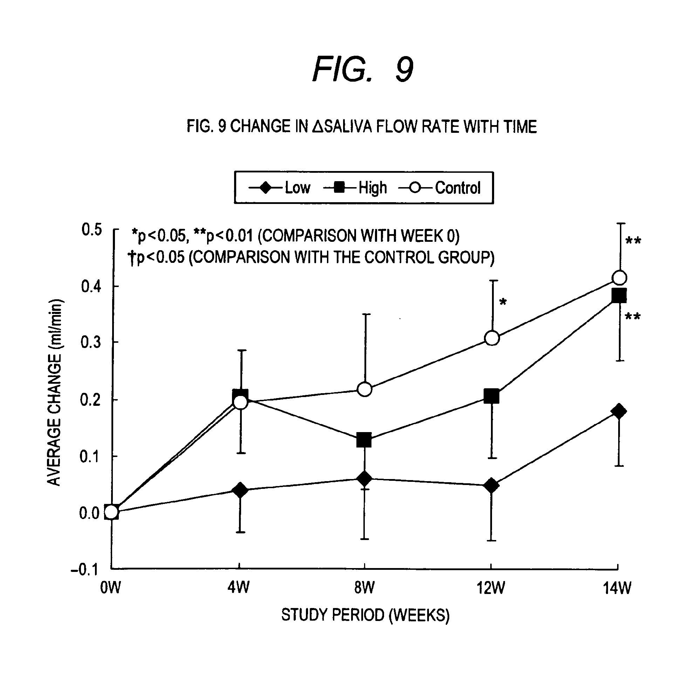

In the saliva flow rate (FIG. 9), a significant increase was observed at week 12 (p < 0.05) and week 14 (p < 0.01) in the control group and at week 14 (p < 0.01) in the high concentration group, as compared with week 0. In addition, no significant difference was observed at weeks 4, 8, 12 and 14 among the control group, the low concentration group and the high concentration group.

### 2. Oral Findings and Questioning

No significant decalcification or coloration of teeth was observed during the study period. In addition, the following oral findings, which seem to be clearly abnormal, were not observed: significant inflammation or ulcer formation in the buccal mucosa, gingiva and tongue. There were no systemic symptoms including diarrhea, which may be seen as a problem in questioning.

Only one subject complained about the coloration of teeth, but the degree of the coloration was generally and commonly seen. Thus, the possibility of the coloration due to the consumption of the study gums seemed to be very low. After completion of the study, full mouth polishing of the tooth surface of the subject was carried out to remove the coloration.

### (Conclusion)

It was shown in the plaque index, the gingival index, the bleeding on probing and the probing pocket depth that the chewing gum incorporating a eucalyptus extract of the present invention possibly has a preventive effect on periodontal disease. It is considered that the reason for this is because the eucalyptus extract eluted from the gum inhibits the accumulation of plaque which causes gingivitis or periodontitis and inhibits gingival inflammation and thus a significant difference was observed in the clinical index.

Further, the chewing gum incorporating a eucalyptus extract of the present invention had a significant difference even in the amount of tongue coating or organoleptic test as compared with the control gum. Since tongue coating or plaque is a main generation source of bad breath, the chewing gum incorporating a eucalyptus extract is also expected to have a preventive effect on bad breath.

### (Example 4)

A lozenge was prepared as follows by using the eucalyptus extract prepared in Example 1.

| | |
|---|---|
| Glucose | 73.8% |
| Lactose | 18.0 |
| Gum Arabic | 6.0 |
| Flavor | 1.0 |
| Sodium Monofluorophosphate | 0.7 |
| Eucalyptus Extract | 0.5 |
| | 100.0% |

### (Example 5)

A candy was prepared as follows by using the eucalyptus extract prepared in Example 1.

| | |
|---|---|
| Sugar | 50.0% |
| Starch Syrup | 33.4 |
| Citric Acid | 1.0 |
| Flavor | 0.2 |
| Eucalyptus Extract | 0.1 |
| Water | 15.3 |
| | 100.0% |

### (Example 6)

A gummy jelly was prepared as follows by using the eucalyptus extract prepared in Example 1.

| | |
|---|---|
| Gelatin | 60.0% |
| Starch Syrup | 23.0 |
| Sugar | 8.0 |
| Vegetable Fat and Oil | 4.5 |
| Mannitol | 3.0 |
| Lemon Juice | 1.0 |
| Eucalyptus Extract | 0.5 |
| | 100.0% |

### (Example 7)

A tablet was prepared as follows by using the eucalyptus extract prepared in Example 1.

| | |
|---|---|
| Sugar | 75.8% |
| Glucose | 19.0 |
| Sucrose Esters of Fatty Acids | 0.2 |
| Flavor | 0.2 |
| Eucalyptus Extract | 0.8 |
| Water | 4.0 |
| | 100.0% |

### (Example 8)

A mouth spray was prepared as follows by using the eucalyptus extract prepared in Example 1.

| | |
|---|---|
| Ethanol | 10.0% |
| Glycerin | 5.0 |
| Flavor | 0.05 |
| Coloring Agents | 0.001 |
| Eucalyptus Extract | 0.005 |
| Water | Balance |
| | 100.0% |

This application claims the priority from Japanese Patent Application No. 2009-105121, filed on April 23, 2009, which is hereby incorporated by reference herein in its entirety.

## Claims

1. An oral composition having a tongue coating removing action, wherein the oral composition comprises an extract of eucalyptus, a plant of the Myrtaceae family, as an essential ingredient.

2. The oral composition according to claim 1, wherein the oral composition is confectionery such as a chewing gum, a candy, a tablet and a gummy jelly, a mouth spray and a mouthwash.

3. The oral composition according to claim 1 or 2, wherein the content of the eucalyptus extract in the oral composition is 0.001% by weight or more and 10.0% by weight or less.

4. An oral composition having a preventive action on bad breath, wherein the oral composition comprises an extract of eucalyptus, a plant of the Myrtaceae family, as an essential ingredient.
